# EUROPEAN PATENT APPLICATION

(11) **EP 3 170 463 A1**
(43) Date of publication of application: **24.05.2017**
(21) Application number: 15821275.3
(22) Date of filing: 08.07.2015
(51) Int. Cl.: A61B 17/3201, A61B 18/00, A61B 18/12

(54) **INSTRUMENT**

(30) Priority: 15.07.2014 JP 2014145307
(71) Applicant: Olympus Corporation, Hachioji-shi Tokyo 192-8507 (JP)
(72) Inventor: AKAGANE, Tsunetaka, Tokyo 192-8507 (JP)
(74) Representative: Gunzelmann, Rainer
(86) International application number: PCT/JP2015/069670
(87) International publication number: WO 2016/009921

(57) **Abstract**

A treatment tool comprising, a probe including a first surface that carries out incision of the living tissue with the ultrasonic vibrations, a second surface that carries out coagulation of the living tissue, and an insulation portion that covers the first surface, and a jaw which is engageable with the probe and disengageable from the probe, the jaw including a third surface facing the first surface in an engaging state and a fourth surface facing the second surface in the engaging state.

## Description

### FIELD

The present invention relates to a treatment tool which treats a living tissue by ultrasonic vibrations.

### BACKGROUND

Jpn. Pat. Appln. KOKAI Publication No. 2009-160404 (Patent Literature 1) discloses a general surgical apparatus. The surgical apparatus may perform surgical procedures, such as incision, resection, or coagulation of a living tissue utilizing ultrasonic waves, and also may perform procedures with high-frequency waves.

### CITATION LIST

### PATENT LITERATURE

Patent Literature 1: Jpn. Pat. Appln. KOKAI Publication No. 2009-160404

### SUMMARY

### TECHNICAL PROBLEM

It is desirable that the treatment tool, such as the surgical apparatus mentioned above, be a so-called minimally-invasive treatment tool, which exerts little effects on tissues of a patient during procedures.

An object of the present invention is to provide a minimally-invasive treatment tool.

### SOLUTION TO PROBLEM

A treatment tool comprising, a probe which is rod-shaped, which forms a first electrode to cause a high-frequency current to flow through a living tissue and to which ultrasonic vibrations are transmitted, the probe including a first surface that carries out incision of the living tissue with the ultrasonic vibrations, a second surface that carries out coagulation of the living tissue, and an insulation portion that covers the first surface, and a jaw which forms a second electrode to cause the high-frequency current to flow through the living tissue, which is engageable with the probe and disengageable from the probe, the jaw including a third surface facing the first surface in an engaging state and a fourth surface facing the second surface in the engaging state.

### ADVANTAGEOUS EFFECTS

With the above configuration, a minimally-invasive treatment tool can be provided.

### BRIEF DESCRIPTION OF THE DRAWINGS

FIG. 1 is a schematic view showing a general configuration of a treatment tool according to a first embodiment.
FIG. 2 is a perspective view showing a distal end portion of a probe and a jaw of the treatment tool shown in FIG. 1.
FIG. 3 is a cross-sectional view taken along line F3-F3 in FIG. 1.
FIG. 4 is a cross-sectional view taken along line F4-F4 in FIG. 1.
FIG. 5 is a cross-sectional view taken along line F5-F5 in FIG. 1.
FIG. 6 is a cross-sectional view showing a state in which a living tissue is sandwiched between the probe and the jaw shown in FIG. 5.
FIG. 7 is a cross-sectional view showing a probe and a jaw of a treatment tool according to a second embodiment.
FIG. 8 is a cross-sectional view showing a state in which a living tissue is sandwiched between the probe and the jaw shown in FIG. 7.
FIG. 9 is a cross-sectional view showing a probe and a jaw of a treatment tool according to a third embodiment.
FIG. 10 is a cross-sectional view showing a state in which a living tissue is sandwiched between the probe and the jaw shown in FIG. 9.

### DETAILED DESCRIPTION

### [First Embodiment]

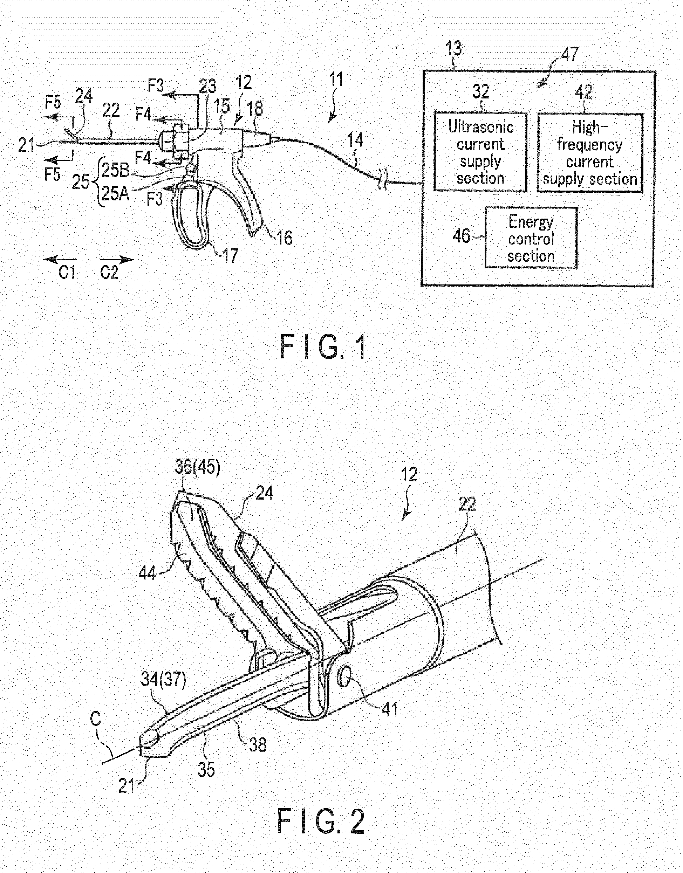

The first embodiment of the present invention will be explained with reference to FIG. 1 to FIG. 6.

As shown in FIG. 1, a treatment tool 11 comprises a hand piece 12, a power supply unit 13, and a cable 14 connecting the hand piece 12 and the power supply unit 13.

As shown in FIGS. 1 and 2, the hand piece 12 comprises a holding section 15 forming a shell, a fixed handle 16 fixed to the holding section 15, a movable handle 17 rotatable with respect to the holding section 15, a vibration generation section 18 (transducer) housed in the holding section 15, a rod-shaped probe 21 connected to the vibration generation section 18, a cylindrical sheath 22 covering a periphery of the probe 21 to protect the probe 21, a knob 23 (rotating knob) fixed to the sheath 22, and a jaw 24 rotatably attached to the probe 21 and the sheath 22. One end of the cable 14 is connected to the holding section 15. The other end of the cable 14 is connected to the power supply unit 13. In this embodiment, one of two directions parallel to a longitudinal direction C of the probe 21 is referred to as a distal direction C1 and a direction opposite to the distal direction is referred to as a proximal direction C2. A cushioning material (elastic material) to absorb vibrations generated from the vibration generation section 18 may be provided between the inner surface of the holding section 15 and the vibration generation section 18.

The holding section 15 is provided with two energy operation input buttons 25. A doctor can apply energy (ultrasonic vibrations and a high-frequency current) via the probe 21 to a living tissue of a subject of treatment by operating the two energy operation input buttons 25. A first energy operation input button 25A corresponds to a so-called coagulation mode, to output ultrasonic vibrations and a high-frequency current suitable for coagulation of a living tissue and sealing of a blood vessel. A second energy operation input button 25B corresponds to a so-called coagulation/incision mode, to output ultrasonic vibrations and a high-frequency current suitable for coagulation and incision of a living tissue or sealing and incision of a blood vessel.

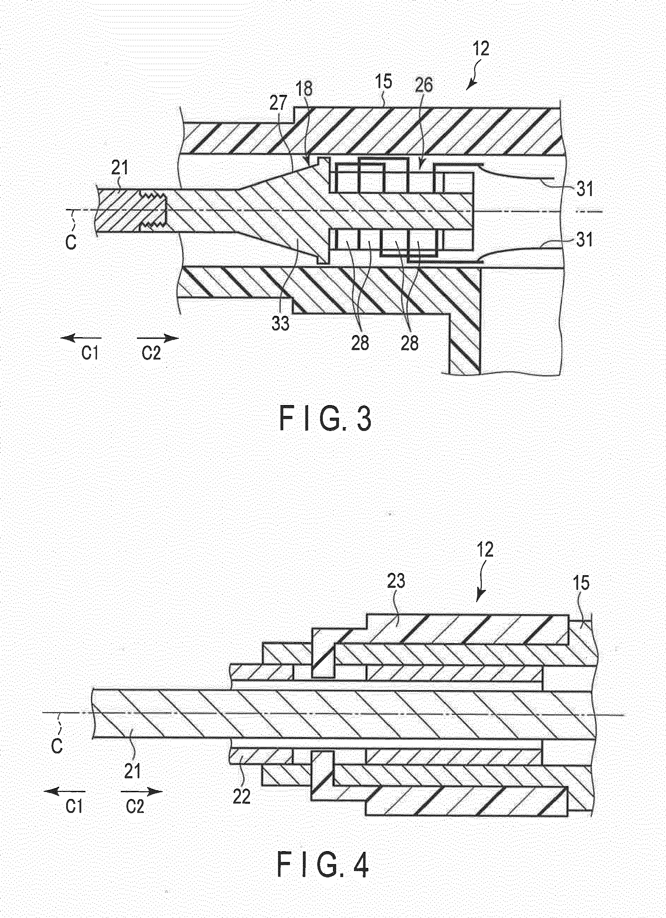

As shown in FIG. 3, the vibration generating section 18 comprises an ultrasonic vibrator 26 and a horn member 27. The ultrasonic vibrator 26 comprises piezoelectric elements 28 (four elements in this embodiment), which change an electric current to ultrasonic vibrations. One end of an electrical wiring 31 is connected to the ultrasonic vibrator 26. The electrical wiring 31 extends inside the cable 14 and connects with an ultrasonic current supply section 32 of the power supply unit 13 at the other end. When electric power is supplied from the ultrasonic current supply section 32 to the ultrasonic vibrator 26 through the electrical wiring 31, the ultrasonic vibrator 26 generates ultrasonic vibrations.

The ultrasonic vibrator 26 is attached to the horn member 27. The horn member 27 is made of a metal material. The horn member 27 has a substantially conical cross-section change portion 33, whose cross section is reduced toward the distal direction C1 of the probe 21. The ultrasonic vibrations generated by the ultrasonic vibrator 26 are transmitted to the horn member 27. In the cross-section change portion 33, amplitudes of the ultrasonic vibrations are increased.

The probe 21 is made of, for example, a biocompatible metal material (e.g., a titanium alloy) and shaped into a rod. A proximal end portion of the probe 21 is connected to one of two second electrical wirings. Ultrasonic vibrations are transmitted from the vibration generation section 18 to the probe 21, and a high-frequency current is supplied from a high-frequency current supply section 42 to the probe 21. Therefore, the probe 21 can not only apply ultrasonic vibrations to a living tissue, but also function as a first electrode (negative electrode) of a bipolar electrosurgical knife.

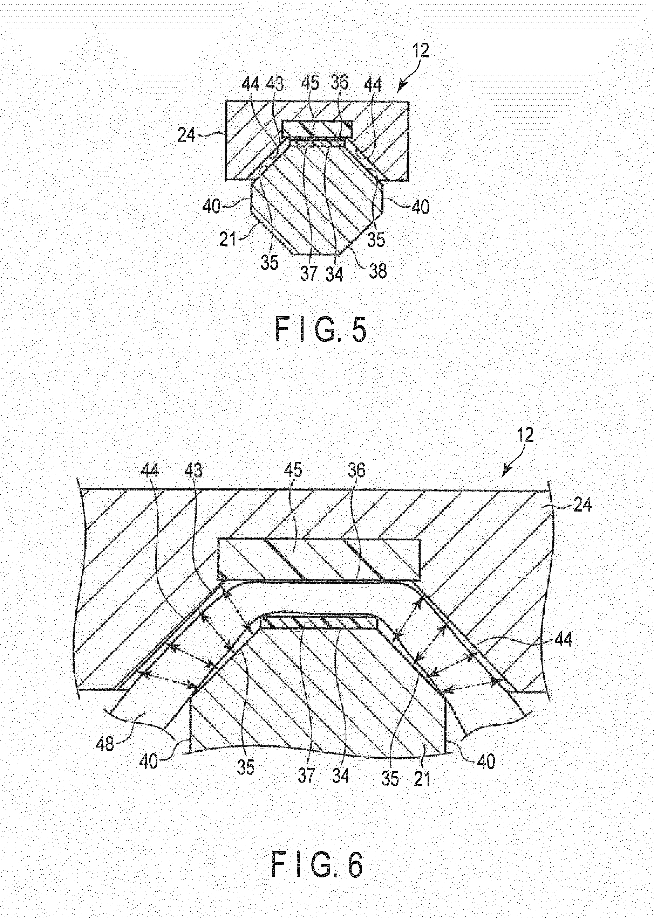

As shown in FIG. 5, the probe 21 has, for example, a polygonal cross section (in this embodiment, an octagonal cross section as an example). The probe 21 comprises a first surface 34 (an incision surface, a contact surface) mainly for use in incision of a living tissue (including a blood vessel, etc.) with ultrasonic vibrations, a second surface 35 (a sealing surface) slanted to the first surface 34, a side surface 40 provided outside of the second surface 35 in a width direction, an insulation portion 37 provided on the first surface 34, and a non-contact portion 38 (a non-contact surface) located on a side opposite to the first surface 34 and the second surface 35. The second surface 35 is mainly for use in coagulation of a living tissue and sealing of a blood vessel. The second surface 35 is provided in two places with the first surface 34 interposed therebetween.

The first surface 34 of the probe 21 is coated with the insulation portion 37 (insulating thin film) made of a synthetic resin material. The insulation portion 37 may be formed to cover the first surface 34 with a thin plate made of a synthetic resin material. For example, polyether ether ketone (PEEK) may be used for a material of the insulation portion 37. The insulating portion 37 may also be made of PTFE or a resin containing a carbon nanotube, or any other lubricant resin material.

As shown in FIG. 2 and FIG. 4, the sheath 22 is cylindrical and protects the probe 21 located inside. The sheath 22 is attached to the holding section 15 to be rotatable relative to the holding section 15 at a proximal end portion. The knob 23 is fixed to the sheath 22 (refer to FIG. 4). The sheath 22 comprises a pin 41 in a distal end portion. The other one of the two second electrical wirings is connected to a proximal end portion of the sheath 22. The sheath 22 and the jaw 24 at the distal end of the sheath form a second electrode (positive electrode) of the bipolar electrosurgical knife. The second electrical wirings each extend inside the cable 14 and connect with the high-frequency current supply section 42 of the power supply unit 13 at the other end.

The jaw 24 is supported by the pin 41 fixed to the distal end portion of the sheath 22, and is attached to be rotatable around the pin 41. The jaw 24 is capable of engaging with the probe 21 to grasp a living tissue and disengaging from the probe 21 by operations of the movable handle 17. The jaw 24 is configured as a plate including a concave portion 43 in a central portion to house the probe 21, so as to engage with the probe 21 having an octagonal cross section. The jaw 24 is made of, for example, a biocompatible metal material (e.g., a titanium alloy).

As shown in FIG. 5, the jaw 24 (the concave portion 43) comprises a third surface 36 that faces the first surface 34 in a state of engaging with the probe 21, and a fourth surface 44 that faces the second surface 35 in a state of engaging with the probe 21. The fourth surface 44 is slanted to the third surface 36. The jaw 24 comprises a platelike insulation member 45 (a third insulation portion) at a position corresponding to the third surface 36. The insulation member 45 covers the third surface 36. The insulation member 45 is also called a tissue pad, and prevents a metal portion of the probe 21 from being brought into direct contact with a metal portion of the jaw 24 in a state where the jaw 24 is engaged with the probe 21. The insulation member 45 is made of a synthetic resin material. For example, polyether ether ketone (PEEK) may be used for a material of the insulation member 45. The insulation member 45 may also be made of PTFE or a resin containing a carbon nanotube, or any other lubricant resin material.

As shown in FIG. 1, the power supply unit 13 comprises the ultrasonic current supply section 32, the high-frequency current supply section 42, and an energy control section 46 that controls these sections. The energy control section 46 can control supply of an ultrasonic generating current from the ultrasonic current supply section 32, and supply of a high-frequency current from the high-frequency current supply section 42. The ultrasonic current supply section 32 and the high-frequency current supply section 42 form an energy generation section 47. When the energy operation input button 25 is operated by the doctor, an electric signal is transmitted to the energy control section 46 and input of an energy operation is detected. As a result, the energy control section 46 supplies an ultrasonic generating current from the ultrasonic current supply section 32 to the probe 21, and supplies a high-frequency current from the high-frequency current supply section 42 to the probe 21.

Functions of the treatment tool 11 of the embodiment will be described with reference to FIG. 5 and FIG. 6. In a state where a living tissue 48 is sandwiched between the probe 21 and the jaw 24, the doctor operates the energy operation input button 25, so that energy can be applied to the living tissue 48. When the energy operation input button 25 corresponding to the coagulation/incision mode (the second energy operation input button 25B) is operated, the probe 21 ultrasonically vibrates and applies thermal energy generated by frictional motion to the living tissue 48. As a result, incision of the living tissue 48 and a blood vessel can be carried out by the first surface 34 of the probe 21 and the third surface 36 of the jaw 24. At the same time, a high-frequency current flows to the living tissue between the second surface 35 of the probe 21 and the fourth surface 44 of the jaw 24 which serve as electrodes, so that electric energy can be applied to the living tissue 48.

Thus, in this embodiment, since two types of energy are applied from the probe 21 and the jaw 24, coagulation and incision of the living tissue 48 sandwiched therebetween can be efficiently carried out. At that time, the first surface 34 is covered by the insulation portion 37. Similarly, the third surface 36 is covered by the insulation member 45 (third insulation portion). With those configurations, the energy of the high-frequency current flowing between the probe 21 and the jaw 24 can be concentrated around the second surface 35 and the fourth surface 44. As a result, the total quantity of energy required for coagulation of the living tissue 48 can be reduced, and the time required for coagulation of the living tissue 48 can be reduced.

Furthermore, the doctor can carry out coagulation of the living tissue 48 by operating the first energy operation input button 25A in the state where the living tissue 48 is sandwiched between the probe 21 and the jaw 24. In this case, the thermal energy generated by ultrasonic vibrations is applied to the living tissue 48 between the first surface 34 of the probe 21 and the third surface 36 of the jaw 24. At the same time, the high-frequency current flows to the living tissue 48 between the second surface 35 of the probe 21 and the fourth surface 44 of the jaw 24. In this case also, the energy of the high-frequency current can be concentrated around the second surface 35 and the fourth surface 44. As a result, the total quantity of energy required for coagulation of the living tissue 48 can be reduced, and the time required for coagulation of the living tissue 48 can be reduced.

According to the first embodiment, the treatment tool 11 comprises: the probe 21 which is rod-shaped, which forms the first electrode to cause a high-frequency current to flow through the living tissue 48 and to which ultrasonic vibrations are transmitted, the probe 21 including the first surface 34 that carries out incision of the living tissue 48 with the ultrasonic vibrations, the second surface 35 that carries out coagulation of the living tissue 48, and the insulation portion 37 that covers the first surface 34; and the jaw 24 which forms the second electrode to cause the high-frequency current to flow through the living tissue 48, which is engageable with the probe 21 and disengageable from the probe 21, the jaw 24 including the third surface 36 facing the first surface 34 in the engaging state and the fourth surface 44 facing the second surface 35 in the engaging state.

With this configuration, the energy density of the high-frequency current can be high at a position between the second surface 35 of the probe 21 and the fourth surface 44 of the jaw 24. As a result, coagulation of the living tissue 48 can be carried out with less energy, and the time required for coagulation of the living tissue 48 can be reduced. Accordingly, it is possible to reduce thermal invasion to the living tissue 48 that results from diffusion of heat due to the high-frequency current to surrounding tissues, thereby reducing the burden on a patient who is undergoing surgery.

The second surface 35 is slanted to the first surface 34, and the fourth surface 44 is slanted to the third surface 36. With this configuration, the living tissue 48 that extends across a portion between the first surface 34 and the third surface 36 and a portion between the second surface 35 and the fourth surface 44 can be curved. As a result, the force pushing the living tissue 48 against the probe 21 or the jaw 24 can be increased in the portion between the first surface 34 and the third surface 36 or the portion between the second surface 35 and the fourth surface 44. Accordingly, frictional force that acts between the living tissue 48 and the probe 21 or the jaw 24 can be increased. Therefore, the living tissue 48 can be prevented from being displaced when the living tissue 48 is coagulated or incised. As a result, the operability in surgery can be improved.

### [Second Embodiment]

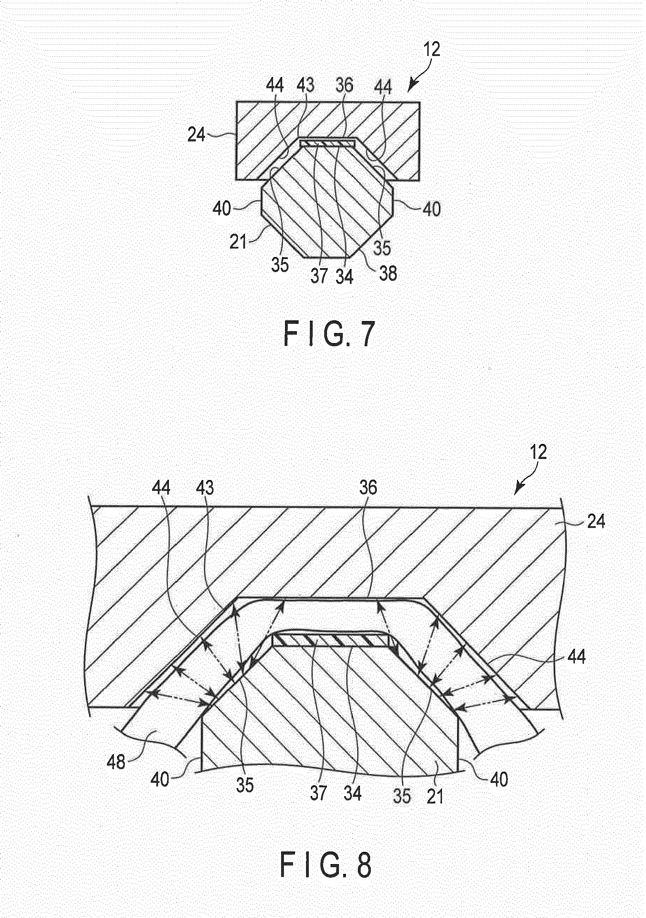

A treatment tool of a second embodiment will be described with reference to FIG. 7 and FIG. 8. The treatment tool 11 of the second embodiment differs from the first embodiment in that an insulation member 45 is not provided in a jaw 24; the other parts are the same as those of the first embodiment. Therefore, mainly those portions different from the first embodiment will be explained. Portions the same as the first embodiment will not be explained or illustrated in the drawings.

The jaw 24 (the concave portion 43) comprises a third surface 36 that faces a first surface 34 in a state of engaging with a probe 21, and a fourth surface 44 that faces a second surface 35 in a state of engaging with the probe 21. In this embodiment, an insulation member 45 is omitted from the third surface 36 of the jaw 24.

Functions of the treatment tool 11 of the embodiment will be described with reference to FIG. 7 and FIG. 8. In the state where a living tissue 48 is sandwiched between the probe 21 and the jaw 24, the doctor operates an energy operation input button 25, so that energy can be applied to the living tissue 48. When an energy operation input button 25 corresponding to the coagulation/incision mode (a second energy operation input button 25B) is operated, the probe 21 ultrasonically vibrates and applies thermal energy generated by frictional motion to the living tissue 48. As a result, incision of the living tissue 48 and a blood vessel can be carried out by the first surface 34 of the probe 21 and the third surface 36 of the jaw 24. At the same time, a high-frequency current flows to the living tissue 48 between the second surface 35 of the probe 21 and the fourth surface 44 of the jaw 24 which serve as electrodes, so that electric energy can be applied to the living tissue 48.

In this embodiment, since the insulation portion 37 is provided on the first surface 34 of the probe 21, the energy of the high-frequency current can be concentrated between the second surface 35 and the fourth surface 44. As a result, the total quantity of energy required for coagulation of the living tissue 48 can be reduced, and the time required for coagulation of the living tissue 48 can be reduced.

Furthermore, the doctor can carry out coagulation of the living tissue 48 by operating a first energy operation input button 25A in the state where the living tissue 48 is sandwiched between the probe 21 and the jaw 24. In this case also, the energy of the high-frequency current can be concentrated around the second surface 35 and the fourth surface 44. As a result, the total quantity of energy required for coagulation of the living tissue 48 can be reduced, and the time required for coagulation of the living tissue 48 can be reduced.

According to this embodiment, the insulation member 45 of the jaw 24 can be omitted, and the structure on the side of the jaw 24 can be simplified. In addition, the energy density of the high-frequency current can be high at a position between the second surface 35 of the probe 21 and the fourth surface 44 of the jaw 24. As a result, coagulation of the living tissue 48 can be carried out with less energy, and the time required for coagulation of the living tissue 48 can be reduced. Accordingly, it is possible to reduce thermal invasion to the living tissue 48 existing near a treatment site, thereby reducing the burden on a patient who is undergoing surgery.

### [Third Embodiment]

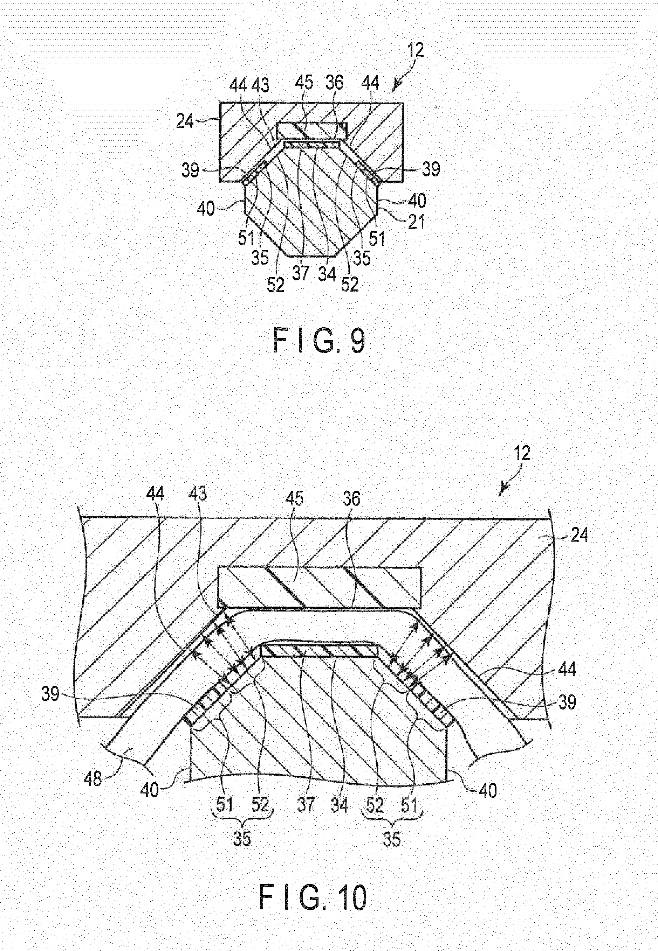

A treatment tool of a third embodiment will be described with reference to FIG. 9 and FIG. 10. The treatment tool of the third embodiment differs from the first embodiment in that a second insulation member 39 is provided on a second surface 35 of a probe 21; the other parts are the same as those of the first embodiment. Therefore, mainly those portions different from the first embodiment will be explained. Portions the same as the first embodiment will not be explained or illustrated in the drawings.

The second surface 35 of the probe 21 includes a first portion 51 provided at a position apart from a first surface 34 and a second portion 52 provided at a position between the first portion 51 and a first surface 34. The first portion 51 occupies, for example, 40 % to 60 % of the area of the second surface 35. The second portion 52 occupies the remainder of the second surface 35. The probe 21 includes a second insulation portion 39 covering the first portion 51.

The first portion 51 of the second surface 35 is coated with the second insulation portion 39 (insulating thin film) made of a synthetic resin material. The second insulation portion 39 may be formed to cover the first portion 51 with a thin plate made of a synthetic resin material. For example, polyether ether ketone (PEEK) may be used for a material of the second insulation member 39. The second insulation portion 39 may also be made of PTFE or a resin containing a carbon nanotube, or any other lubricant resin material.

Functions of a treatment tool 11 of the embodiment will be described with reference to FIG. 9 and FIG. 10. In the state where a living tissue 48 is sandwiched between the probe 21 and a jaw 24, a doctor operates an energy operation input button 25, so that energy can be applied to the living tissue. When the energy operation input button 25 corresponding to the coagulation/incision mode (a second energy operation input button 25B) is operated, the probe 21 ultrasonically vibrates and applies thermal energy to the living tissue 48. As a result, incision of the living tissue 48 and a blood vessel can be carried out by the first surface 34 of the probe 21 and a third surface 36 of the jaw 24. At the same time, a high-frequency current flows to the living tissue 48 between the second portion 52 of the second surface 35 of the probe 21 and a fourth surface 44 of the jaw 24 which serve as electrodes, so that electric energy can be applied to the living tissue 48.

In this embodiment, since the second insulation portion 39 is provided on the first portion 51 of the second surface 35, in addition to the insulation portion 37 on the first surface 34 of the probe 21, the energy of the high-frequency current can be further concentrated between the second portion 52 of the second surface 35 and the fourth surface 44. As a result, the total quantity of energy required for coagulation of the living tissue 48 can be reduced, and the time required for coagulation of the living tissue 48 can be reduced.

Furthermore, the doctor can carry out coagulation of the living tissue 48 by operating a first energy operation input button 25A in the state where the living tissue 48 is sandwiched between the probe 21 and the jaw 24. In this case also, the energy of the high-frequency current can be concentrated around the second portion 52 of the second surface 35 and the fourth surface 44. As a result, the total quantity of energy required for coagulation of the living tissue 48 can be reduced, and the time required for coagulation of the living tissue 48 can be reduced.

According to the third embodiment, the second surface 35 includes the first portion 51 provided at a position apart from the first surface 34 and the second portion 52 provided at a position between the first portion 51 and the first surface 34. The probe 21 includes the second insulation portion 39, and the second insulation portion 39 covers the first portion 51 of the second surface 35.

With this configuration, the energy density of the high-frequency current can be high at a position between the second portion 52 of the second surface 35 of the probe 21 and the fourth surface 44 of the jaw 24. As a result, coagulation of the living tissue can be carried out with less energy, and the time required for coagulation of the living tissue 48 can be reduced. Accordingly, it is possible to reduce thermal invasion to the living tissue 48 existing near a treatment site, thereby reducing the burden on a patient who is undergoing surgery.

The present invention is not limited to the embodiments described above, and various modifications may be made without departing from the gist of the invention. Furthermore, it is natural that a treatment tool may be formed by combining any of the treatment tools of the embodiments described above.

## Claims

1. A treatment tool comprising:
a probe which is rod-shaped, which forms a first electrode to cause a high-frequency current to flow through a living tissue and to which ultrasonic vibrations are transmitted, the probe including a first surface that carries out incision of the living tissue with the ultrasonic vibrations, a second surface that carries out coagulation of the living tissue, and an insulation portion that covers the first surface; and
a jaw which forms a second electrode to cause the high-frequency current to flow through the living tissue, which is engageable with the probe and disengageable from the probe, the jaw including a third surface facing the first surface in an engaging state and a fourth surface facing the second surface in the engaging state.

2. The treatment tool according to claim 1, wherein:
the second surface includes a first portion provided at a position apart from the first surface and a second portion provided at a position between the first portion and the first surface; and
the probe includes a second insulation portion, and the second insulation portion covers the first portion of the second surface.

3. The treatment tool according to claim 2, wherein the jaw includes a third insulation portion covering the third surface.

4. The treatment tool according to claim 3, wherein the second surface is slanted to the first surface, and the fourth surface is slanted to the third surface.
